# EUROPEAN PATENT APPLICATION

(11) **EP 4 644 400 A1**
(43) Date of publication of application: **05.11.2025**
(21) Application number: 23912679.0
(22) Date of filing: 13.12.2023
(51) Int. Cl.: C07H 3/02, C07H 1/00

(54) **CRYSTALLINE ALLULOSE**

(30) Priority: 28.12.2022 KR 20220187868
(71) Applicant: Samyang Corporation, Jongno-gu Seoul 03129 (KR)
(72) Inventor: KIM, Goeun, Seongnam-si Gyeonggi-do 13434 (KR); KIM, Minjeong, Yongin-si, Gyeonggi-do 16823 (KR); RYU, Kyunghun, Seongnam-si, Gyeonggi-do 13340 (KR); PARK, Jiwon, Seongnam-si, Gyeonggi-do 13544 (KR); KIM, Dohyun, Seongnam-si, Gyeonggi-do 13504 (KR); KIM, Minyoung, Suwon-si, Gyeonggi-do 16577 (KR); SA, Soonok, Yongin-si, Gyeonggi-do 16923 (KR); HAN, Jungsook, Anyang-si, Gyeonggi-do 14062 (KR)
(74) Representative: Dr. Schön, Neymeyr & Partner Patentanwälte mbB
(86) International application number: PCT/KR2023/020495
(87) International publication number: WO 2024/144004

(57) **Abstract**

The present application relates to: crystalline allulose exhibiting an X-ray powder diffraction (XRD) pattern in which peaks appear at 20 diffraction angles of 18.8±0.5°, 15.2±0.5°, and 19.5±0.5° in an XRD analysis; a sweetener composition containing the crystalline allulose; and a method for preparing the crystalline allulose.

## Description

### [TECHNICAL FIELD]

The present disclosure relates to an allulose crystal.

### [BACKGROUND ART]

Allulose is a functional sweetener, and it can substitute sugar or fructose or the like, but there are problems that it is difficult to prepare allulose in a uniform and crystalline form due to low crystallinity, and when it is prepared in a crystalline form, a caking phenomenon occurs due to rapid changes in temperature and humidity during storing allulose crystals. In particular, in case of paper bag packaging in which allulose crystals are loaded, there are problems that a surface caking phenomenon in which crystal particles flocculate with each other under moisture and pressure in the atmosphere can further accelerate, and the caking hardness further increases during long-term loading when the surface caking begins. Such high caking hardness makes use of products very limited.

Accordingly, a novel allulose crystal with improved usability is needed in which caking phenomenon during long-term storage is inhibited, and having low hygroscopicity and excellent flowability.

### [DISCLOSURE]

### [TECHNICAL PROBLEM]

One embodiment of the present disclosure is to provide an allulose crystal having a specific diffraction angle pattern.

Another embodiment of the present disclosure is to provide a sweetener composition comprising an allulose crystal having a specific diffraction angle pattern.

Other embodiment of the present disclosure is to provide a method of preparing an allulose crystal having a specific diffraction angle pattern, comprising adding seeds to allulose solution by controlling the total specific surface area of all seeds contained in the crystallization reaction system; and forming an allulose crystal while controlling the degree of supersaturation of the allulose solution.

### [TECHNICAL SOLUTION]

One embodiment of the present disclosure relates to an allulose crystal, having an X-ray powder diffraction pattern comprising peaks at positions of 2θ diffraction angles of 18.8+0.5°, 15.2+0.5°, and 19.5±0.5° in X-ray powder diffraction (XRD) analysis.

Another embodiment of the present disclosure relates to a sweetener composition comprising the allulose crystal according to one embodiment of the present disclosure.

Other embodiment of the present disclosure relates to a method of preparing the allulose crystal according to one embodiment of the present disclosure, comprising a step of adding seeds to allulose solution so that the total specific surface area of all seeds contained in the crystallization reaction system is 0.05 m²/100g or less; and a step of forming an allulose crystal while maintaining the degree of supersaturation of the allulose solution at 1.15 or less.

Hereinafter, the present invention will be described in more detail.

The allulose crystal according to one embodiment of the present disclosure may have an X-ray powder diffraction pattern comprising peaks at positions of 20 diffraction angles of 18.8±0.5°, 15.2±0.5°, and 19.5±0.5° in X-ray powder diffraction (XRD) analysis. Specifically, the allulose crystal may have an X-ray powder diffraction pattern comprising peaks at positions of 2θ diffraction angles of 18.8±0.2°, 15.2±0.2°, and 19.5±0.2° in X-ray powder diffraction (XRD) analysis.

As one embodiment, the allulose crystal may have an X-ray powder diffraction pattern comprising peaks of 2θ diffraction angles of 18.8±0.5°, 15.2±0.5°, 19.5±0.5°, and 28.4±0.5°; or 18.8±0.5°, 15.2±0.5°, 19.5±0.5°, and 20.3±0.5° in X-ray powder diffraction (XRD) analysis.

As one embodiment, the allulose crystal may have an X-ray powder diffraction pattern comprising peaks of 2θ diffraction angles of 18.8±0.2°, 15.2±0.2°, 19.5±0.2°, and 28.4±0.2°; or 18.8±0.2°, 15.2±0.2°, 19.5±0.2°, and 20.3±0.2° in X-ray powder diffraction (XRD) analysis.

The peaks may be peaks with a relative intensity of 5% or more, 6% or more, 7% or more, 10% or more, 11% or more, 12% or more, 15% or more, 17% or more, 18% or more, 20% or more, 25% or more, 30% or more, 34% or more, 35% or more, 40% or more, 45% or more, 50% or more, 60% or more, 65% or more, 70% or more, 75% or more, 80% or more, 85% or more, 90% or more, or 95% or more in the X-ray powder diffraction (XRD) analysis result. The relative intensity shows an intensity of each peak as a relative numerical percentage, based on 100% of the intensity of the peak with the maximum intensity.

Among the peaks, several peaks from the highest relative intensity, or peaks with a relative intensity above a certain value may be major peaks that determine a crystalline form. For example, the major peaks may be 1 to 5, 1 to 4, 1 to 3, or 1 to 2 peaks with the highest relative intensity. For example, the major peaks may be peaks with a relative intensity of 50% or more, 55% or more, or 60% or more.

Therefore, the diffraction angle of the allulose crystal described above may be an diffraction angle at a major peak with a high relative intensity in X-ray powder diffraction (XRD) analysis, and the order of description of the diffraction angle may be described from the diffraction angle with the highest relative intensity in X-ray powder diffraction (XRD) analysis.

As one embodiment, the allulose crystal may have an X-ray powder diffraction pattern comprising peaks at positions of 2θ diffraction angles of 18.8±0.5°, 15.2±0.5°, and 19.5±0.5° in the order from the peak with the higher relative intensity to the peak with the lower relative intensity in the X-ray powder diffraction (XRD) analysis. Specifically, the allulose crystal may have an X-ray powder diffraction pattern comprising characteristic peaks at positions of 2θ diffraction angles of 18.8±0.2°, 15.2±0.2°, and 19.5±0.2° in the order from the peak with the higher relative intensity to the peak with the lower relative intensity in the X-ray powder diffraction (XRD) analysis.

As one embodiment, the allulose crystal may have an X-ray powder diffraction pattern comprising peaks at positions of 2θ diffraction angles of 18.8±0.5°, 15.2±0.5°, 19.5±0.5°, and 28.4±0.5°, in the order from the peak with the higher relative intensity to the peak with the lower relative intensity in the X-ray powder diffraction (XRD) analysis. Specifically, the allulose crystal may have an X-ray powder diffraction pattern comprising peaks at positions of 2θ diffraction angles of 18.8±0.2°, 15.2±0.2°, 19.5±0.2°, and 28.4±0.2°, in the order from the peak with the higher relative intensity to the peak with the lower relative intensity in the X-ray powder diffraction (XRD) analysis.

As one embodiment, the allulose crystal may have an X-ray powder diffraction pattern comprising peaks at positions of 2θ diffraction angles of 18.8±0.5°, 15.2±0.5°, 19.5±0.5°, and 20.3±0.5° in the order from the peak with the higher relative intensity to the peak with the lower relative intensity in the X-ray powder diffraction (XRD) analysis. Specifically, the allulose crystal may have an X-ray powder diffraction pattern comprising peaks at positions of 2θ diffraction angles of 18.8±0.2°, 15.2±0.2°, 19.5±0.2°, and 20.3±0.2° in the order from the peak with the higher relative intensity to the peak with the lower relative intensity in the X-ray powder diffraction (XRD) analysis.

As one embodiment, the allulose crystal may have an X-ray powder diffraction pattern in which the peak with the highest relative intensity is positioned at 2θ angles of diffraction of 18.8±0.5° or 18.8±0.2° in the X-ray powder diffraction (XRD) analysis.

The allulose crystal according to one embodiment of the present disclosure has a low specific surface area, and has a high tap density, and therefore, friction between particles is reduced, so the particles move more easily, and volume decreases more easily when being tapped, so it is advantageous for storage and distribution.

The allulose crystal according to one embodiment of the present disclosure has a low increase rate of the surface caking hardness and a low hygroscopic rate, so it has high storage stability. In case of the product surface in which a packaging material and a product are in contact in a packaged product, a caking effect of the surface may occur due to rapid changes in temperature and humidity depending on storage conditions. In particular, in case of loaded paper bag packaging, caking may be further accelerated, and once surface caking begins, the caking hardness can be further strengthened during long-term loading. In the Example of the present disclosure, as a result of measuring the increase rate of the caking hardness and hygroscopic rate by reproducing a situation at which the allulose crystal according to one embodiment of the present disclosure is exposed to a common storage condition or influence of outdoor air, it has lower caking hardness and hygroscopic rate than a conventional allulose crystal.

Furthermore, the allulose crystal according to one embodiment of the present disclosure has a high mean particles diameter and a low angle of repose, so crystal particles are not accumulated or stagnated and have good flowability, and therefore, pipe transfer is easy in a manufacturing process, and losses in dehydrating/drying/cooling process lines can be reduced. In addition, production speed is improved in a packaging process, and the frequency of packaging defects is decreased, so the productivity and yield can be improved throughout the production.

The crystal form may vary depending on the diffraction angle and relative intensity and the like of peaks in the XRD analysis result, and accordingly, the angle of repose of the allulose crystal may be affected. For example, even if they are peaks in which 2θ angles of diffraction are at same positions in X-ray powder diffraction (XRD) analysis, considering the relative intensity of the peaks together, it may result in a difference in angle of repose depending on the difference in orientation of crystals or crystal form.

The allulose crystal according to the present invention may have a low angle of repose, and for example, the angle of repose of the allulose crystal may be 48°or less, 47°or less, 46°or less, 45°or less, 44°or less, 43°or less, or 42.5° or less, and specifically, it may be 37° to 48°, 37° to 47°, 37° to 46°, 37° to 45°, 37° to 44°, 37° to 43.5°, 37° to 43°, 37° to 42.5°, 40° to 48°, 40° to 47°, 40° to 46°, 40° to 45°, 40° to 44°, 40° to 43.5°, 40° to 43°, or 40° to 42.5°. In addition, the angle of repose of the allulose crystal may be 120% or less, 115% or less, 110% or less, 108% or less, 107% or less, or 106% or less, based on 100% of the angle of repose of sugar, and it may be at a similar level to sugar. At this time, the lower limit of the angle of repose of the allulose crystal based on 100% of the angle of repose of sugar may be for example, 95% or more, 96% or more, 97% or more, 98% or more, 99% or more, 100% or more, more than 100%, 101% or more, 102% or more, 103% or more, 104% or more, or 105% or more, but not limited thereto. A low angle of repose indicates a low angle of stacking of powder, and means high flowability. Therefore, the allulose crystal according to one embodiment of the present disclosure has a uniform shape of particles, so it has an effect of excellent flowability.

Specifically, the allulose crystal according to one embodiment of the present disclosure may have at least one characteristic selected from the group consisting of the following (1) to (5):
(1) a specific surface area of 0.04 m²/g or less, 0.035 m²/g or less, 0.03 m²/g or less, or 0.025 m²/g or less,
(2) a volume-average particle diameter (D[4,3]) of 230 µm or more, 240 µm or more, 250 µm or more, 260 µm or more, 270 µm or more, 280 µm or more, 290 µm or more, 300 µm or more, 310 µm or more, 320 µm or more, 330 µm or more, 340 µm or more, 350 µm or more, 360 µm or more, or 370 µm or more,
(3) an angle of repose of 48°or less, 47°or less, 46°or less, 45°or less, 44°or less, 43°or less, or 42.5°or less,
(4) a hygroscopic rate of 24% or less, 23% or less, 22% or less, or 21.5% or less when stored for 15 hours under conditions of a temperature of 25°C and a humidity of 70%, and
(5) an increase rate in hardness of 40% or less, 35% or less, 30% or less, 25% or less, or 20% or less, after being loaded and stored under conditions of a temperature of 40°C and a humidity of 70% for 0.5 hours, and left at a temperature of 25°C for 30 minutes, and applied a pressure of the same weight as the allulose crystal for 30 minutes.

The allulose crystal according to one embodiment of the present disclosure may be crystallized under a condition that the total specific surface area of all seeds contained in the crystallization reaction system is 0.05 m²/100g or less, 0.045 m²/100g or less, 0.04 m²/100g or less, 0.035 m²/100g or less, or 0.03 m²/100g or less.

The total specific surface area of all seeds present in the crystallization reaction system, may be obtained by multiplying the mean specific surface area value of the seed particles (m²/g) and the seed input (g/100g). The total specific surface area of all seeds may be set by adjusting one or more of the mean specific surface area values of the seed particles and seed input. According to the Example of the present disclosure, it was confirmed that the mean diameter of the allulose crystal was stably larger due to the total specific surface area of all seeds in the crystallization reaction system, than the difference in particle size of the seeds added during preparation of the allulose crystal, and an allulose crystal having a specific diffraction angle pattern was prepared due to stacking and differences in shape of particles formed during the crystallization step, and the constitution of the major peak of the X-ray diffraction angle was different from that of a conventional allulose crystal.

The allulose crystal according to one embodiment of the present disclosure may be crystallized under a condition that the degree of supersaturation of the allulose solution is 1.15 or less, specifically, more than 1 to 1.15 or less, or 1.01 or more to 1.15 or less. According to the Example of the present disclosure, during preparation of an allulose crystal, in the case that the degree of supersaturation of the allulose solution is more than 1.15, and in the case that the degree of supersaturation is maintained at 1.15 or less, the X-ray powder diffraction pattern of the prepared allulose crystal was different. Accordingly, the allulose crystal according to one embodiment of the present disclosure is crystallized under a condition that the degree of supersaturation of the allulose solution is 1.15 or less, and thus, it may have an X-ray powder diffraction pattern comprising peaks of 2θ angles of diffraction of 18.8±0.5°, 15.2±0.5°, and 19.5±0.5° in X-ray powder diffraction (XRD) analysis.

Other embodiment of the present disclosure relates to a method of preparing an allulose crystal, comprising a step of adding seeds to allulose solution so that a total specific surface area of all seeds contained in the crystallization reaction system is 0.05 m²/100g or less; and a step of forming an allulose crystal while maintaining the degree of supersaturation of the allulose solution at 1.15 or less.

The allulose solution for preparing an allulose crystal is a high-purity allulose solution with a high allulose content, and may have an allulose content of 80 % by weight or more, 85 % by weight or more, 90 % by weight or more, 91 % by weight or more, 92 % by weight or more, 93 % by weight or more, 94 % by weight or more, or 95 % by weight or more based on 100 % by weight of the solid content. In addition, the allulose solution may be an allulose solution with solids of 80% or more, 85% or more, or 85% or more, concentrated for crystallization.

The step of forming an allulose crystal may be performed by cooling the temperature of the allulose solution at a speed of -0.5°C/hr or less, -0.4°C/hr or less, or -0.3°C/hr or less. At this time, the step of forming an allulose crystal, may control the cooling speed to control the degree of supersaturation of the allulose solution, and for example, in order to maintain the degree of supersaturation of the allulose solution at 1.15 or less, a step of controlling the speed of cooling of the allulose solution may be included at least once or more.

The maintaining the degree of supersaturation of the allulose solution at 1.15 or less, may control the cooling speed or allulose solid content of the allulose solution. The controlling the cooling speed may specifically comprise deceleration section of the cooling speed, and for example, it may comprise controlling the cooling speed to 0°C/hr, that is, stopping cooling. The stopping cooling may stop cooling until the degree of supersaturation of the allulose solution becomes 1.15 or less. Accordingly, the controlling the cooling speed of the allulose solution may temporarily stop cooling of the allulose solution. Specifically, it may temporarily stop cooling of the allulose solution to maintain the temperature of the allulose solution constantly.

Accordingly, in one embodiment of the present disclosure, the step of forming an allulose crystal while maintaining the degree of supersaturation of the allulose solution at 1.15 or less, or controlling the cooling speed of the allulose solution, so that the degree of supersaturation of the allulose solution is maintained at 1.15 or less, may comprise a first cooling section, a deceleration section of the cooling speed, and a second cooling section, and the deceleration section of the cooling speed may be comprised at least once or more, and it may be performed until the degree of supersaturation of the allulose solution becomes 1.15 or less. Specifically, the first cooling section may cool the allulose solution at a constant rate, and the cooling speed may be reduced in the deceleration section of the cooling speed, and when the degree of supersaturation of the allulose solution becomes 1.15 or less, the second cooling section may be performed. The cooling speed of the first cooling section and the second cooling section may be same or different.

For example, the controlling the speed of cooling of the allulose solution, may be temporarily stopping cooling within a range of the temperature of the allulose solution of 15 to 45°C, 15 to 40°C, 15 to 39°C, 15 to 38°C, 20 to 45°C, 20 to 40°C, 20 to 39°C, 20 to 38°C, 25 to 45°C, 25 to 40°C, 25 to 39°C, 25 to 38°C, 30 to 45°C, 30 to 40°C, 30 to 39°C, 30 to 38°C, 35 to 45°C, 35 to 40°C, 35 to 39°C, 35 to 38°C, 36 to 45°C, 36 to 40°C, 36 to 39°C, 36 to 38°C, 37 to 45°C, 37 to 40°C, 37 to 39°C, or 37 to 38°C. At this time, the temperature of the allulose solution may be maintained constantly within the above range.

Specifically, the step of forming an allulose crystal may comprise a step of maintaining the temperature of the allulose solution by stopping cooling until the degree of supersaturation of the allulose solution reach 1.1 or less, when the degree of supersaturation of the allulose solution is more than 1.15.

The step of forming an allulose crystal, may start cooling of the allulose solution within a range of the degree of supersaturation of the allulose solution of more than 1 to 1.15 or less, or 1.01 to 1.15.

The step of forming an allulose crystal, may starts cooling of the allulose solution at a temperature of 25 to 50°C, 30 to 50°C, 35 to 50°C, 40 to 50°C, 25 to 45°C, 30 to 45°C, 35 to 45°C, or 40 to 45°C.

The step of forming an allulose crystal, may terminate cooling of the allulose solution at a temperature of the allulose solution of 15 to 25°C, 15 to 23°C, 15 to 20°C, 18 to 25°C, 18 to 23°C, or 18 to 20°C.

### [ADVANTAGEOUS EFFECTS]

The allulose crystal according to one embodiment of the present disclosure has different constitutions of X-ray spectroscopic spectra, and the final product has particles with a specific surface area value of 0.04 m²/g or less, diameter of 230 µm or more, and a relatively uniform crystal shape with improved transparency, and has improved hygroscopicity and flowability, and has improved ease of use and a reduced degree of caking during storage, and stability has been improved during packaging, storage, transportation and distribution of the product and usability during long-term storage. In addition, the crystal itself can reduce bitterness and off-taste previously felt after sweetness and achieve mouthfeel similar to sugar.

### [BRIEF DESCRIPTION OF THE DRAWINGS]

FIG. 1a shows appearance of the allulose crystals prepared without controlling the degree of supersaturation and cooling speed of the allulose solution, and FIG. 1b shows appearance of the allulose crystals according to one embodiment of the present disclosure.
FIG. 2 is a graph showing the hygroscopic rate of the allulose crystal according to one embodiment of the present disclosure.
FIG. 3 is a graph showing the change rate in caking hardness of the allulose crystal according to one embodiment of the present disclosure.
FIG. 4 is a sweetness profile graph of the sensory evaluation of the allulose crystal according to one embodiment of the present disclosure.

### [MODE FOR INVENTION]

Hereinafter, the present disclosure will be described in more detail by the following Examples. However, these Examples are intended to illustrate the present disclosure only, but the scope of the present disclosure is not limited by these Examples.

### Comparative Example 1. Preparation of allulose crystals (1)

High-performance liquid chromatography (HPLC) analysis for allulose content analysis was conducted by setting Biorad carbohydrate Aminex-HPX 87C column at a temperature of 80°C and then setting a mobile phase to distilled water (DW) 0.6ml/min using an RI detector.

A high-purity allulose solution with purity of 95 % by weight was concentrated at a concentration with a solid content of 87.2%, and the temperature of the allulose solution was cooled starting from 35°C to 10°C at a speed of -1°C/hr to form allulose crystals. At this time, the degree of supersaturation of the initial crystallization solution was about 1.5, and allulose seeds were not added. The mother liquid was removed by centrifugal dehydration and the allulose crystals were washed with cooling water, and then dried to collect the allulose crystals. The mean particle diameter of the primary obtained allulose crystals was 225µm.

The obtained allulose crystals were crushed by Hammer mill equipment to use them as seeds in the following Example. The mean particle diameter of the allulose crystals of Comparative example 1 was 62.5 µm, and the mean specific surface area was 0.327 m²/g.

**[Table 1]**

| Classification | Mean particle diameter (µm) (Vol. Weighted Mean D[4,3]) | Mean specific surface area value (m²/g) |
|---|---|---|
| Comparative example 1 | 62.5 | 0.3270 |
| Comparative example 2 | 154.0 | 0.0558 |
| Comparative example 3 | 165.6 | 0.0513 |
| Comparative example 4 | 227.8 | 0.0410 |
| Example 1 | 282.7 | 0.0298 |
| Example 2 | 378.4 | 0.0203 |

### Comparative Example 2. Preparation of allulose crystals (2)

A high-purity allulose solution isolated with purity of 95 % by weight was concentrated at a concentration with a solid content of 85%, and the temperature of the solution in a crystallization reactor was maintained at 50°C, and seed with a mean particle diameter of 98.0 µm, and a mean specific surface area of 0.196 m²/g were mixed with ethanol and added at a concentration of 0.3% and then distributed evenly. At this time, the degree of supersaturation of the initial crystallization solution was 1.39, and for crystallization, the cooling temperature was cooled to 30°C at a speed of -1°C/hr per hour. The mother liquid was removed by centrifugal dehydration and the crystals were washed with cooling water, and then dried to collect the obtained allulose crystals.

The physical properties of the prepared allulose crystals were described in Table 1. The particle size of the allulose crystals of Comparative Example 2 was a mean particle diameter of 154.04µm, and the mean specific surface area was 0.0558 m²/g.

### Comparative Example 3. Preparation of allulose crystals (3)

A high-purity allulose solution with purity of 95 % by weight was concentrated at a concentration with a solid content of 86.1%, and the temperature of the concentrated allulose solution was adjusted to 44.5°C so as to set the degree of supersaturation of the allulose solution to 1.05. The crushed crystals prepared in Comparative Example 1 were added to the allulose solution as seeds at a concentration of 1.7 % by weight and stirred to distribute them evenly, and then a crystallization process was prepared. At this time, the total specific surface area of the added seeds was 0.327 m²/g X 1.7 g/100g ≒ 0.556 m²/100g.

After that, allulose crystals were generated while cooling the temperature of the allulose solution constantly at a speed of -0.3°C/hr. At this time, the controlling of cooling speed in order to maintain the degree of supersaturation of the allulose solution constantly was not performed, and cooling was performed at a constant speed until the final temperature reached 20°C. During the crystallization process, the degree of supersaturation increased up to 1.19. The mother liquid was removed by centrifugal dehydration and the crystals were washed with cooling water, and then dried to collect the allulose crystals.

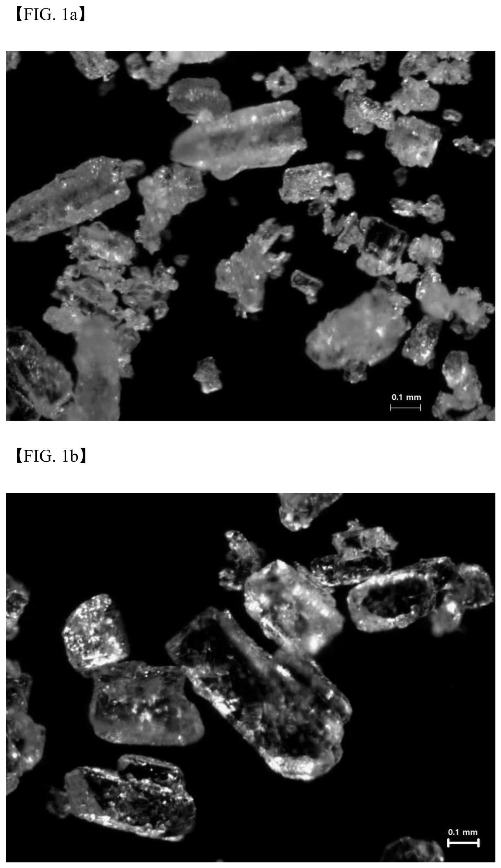

The physical properties of the prepared allulose crystals were described in Table 1. The mean particle diameter of the allulose crystals of Comparative Example 3 was 165.6µm, and the specific surface area value was 0.0513 m²/g. The appearance of the prepared allulose crystals was shown in FIG. 1a.

### Comparative Example 4. Preparation of allulose crystals (4)

A high-purity allulose solution with purity of 95 % by weight was concentrated at a concentration with a solid content of 86.3%, and the temperature of the concentrated allulose solution was adjusted to 45°C so as to set the degree of supersaturation of the allulose solution to 1.057. The allulose crystals having purity of 99%, a mean diameter of 189.5 µm, and a mean specific surface area of 0.040 m²/g were added to the allulose solution as seeds at a concentration of 2.0 % by weight and stirred to distribute them evenly, and then a crystallization process was prepared. The total specific surface area of the seeds was 0.040 m²/g X 2.0 g/100g ≒ 0.080 m²/100g.

After that, allulose crystals were generated while cooling the temperature of the allulose solution constantly at a speed of -0.3°C/hr. At this time, the controlling of cooling speed in order to maintain the degree of supersaturation of the allulose solution constantly was not performed, and cooling was performed at a constant speed until the final temperature reached 20°C. In the crystallization process, the degree of supersaturation increased up to 1.213. The mother liquid was removed by centrifugal dehydration and the crystals were washed with cooling water, and then dried to collect the allulose crystals.

The physical properties of the prepared allulose crystals were described in Table 1. The mean diameter of the allulose crystals of Comparative example 4 was 227.8µm, and the specific surface area value was 0.0410 m²/g.

### Example 1. Preparation of allulose crystals (5)

A high-purity allulose solution with purity of 95 % by weight was concentrated at a concentration with a solid content of 85.4%, and the temperature of the concentrated allulose solution was adjusted to 44.2°C, so as to set the degree of supersaturation of the allulose solution to 1.011. The allulose crystals having purity of 99%, a mean diameter was 189.5 µm, and a mean specific surface area of 0.040 m²/g were added to the allulose solution as seeds at a concentration of 1.0 % by weight and stirred to distribute them evenly, and then a crystallization process was prepared. At this time, the total specific surface area sum of the seeds was 0.040 m²/g X 1.0 g/100g ≒ 0.040 m²/100g.

After that, allulose crystals were generated while cooling the temperature of the allulose solution constantly at a speed of -0.3°C/hr. At this time, in order to maintain the degree of supersaturation of the allulose solution at 1.15 or less, the supernatant was taken from the allulose solution and the concentration was measured, and then cooling was stopped around 38°C where the degree of supersaturation became 1.145, and the constant temperature was maintained until the degree of supersaturation reached 1.1 or less, and then cooling was performed again after checking that the value of the degree of supersaturation of the supernatant was reduced to 1.082. After that, while the temperature of the allulose solution was cooled to 20°C, the degree of supersaturation increased only up to 1.131, and when the degree of supersaturation no longer increased, the crystallization reaction was terminated. The mother liquid was removed by centrifugal dehydration and the crystals were washed with cooling water, and then dried to collect the allulose crystals.

The physical properties of the prepared allulose crystals were described in Table 1. The mean diameter of the allulose crystals of Example 1 was 282.7 µm, and the specific surface area value was 0.0298 m²/g. The allulose crystals in this Example showed relatively uniform particle distribution and a lower specific surface area value compared to Comparative Example 4.

### Example 2. Preparation of allulose crystals (6)

A high-purity allulose solution with purity of 95 % by weight was concentrated at a concentration with a solid content of 85.6%, and the temperature of the concentrated allulose solution was adjusted to 44.7°C and the degree of supersaturation of the allulose solution was set to 1.01. The allulose crystals prepared in Comparative Example 2 were added to the allulose solution as seeds at a concentration of 0.5 % by weight and stirred to distribute them evenly, and then a crystallization process was prepared. The total specific surface area sum of the seeds was 0.0558 m²/g X 0.5 g/100g ≒ 0.0279 m²/100g.

After that, allulose crystals were generated while cooling the temperature of the allulose solution constantly at a speed of -0.3°C/hr. At this time, in order to maintain the degree of supersaturation of the allulose solution at 1.15 or less, the supernatant was taken from the allulose solution and the concentration was measured, and then cooling was stopped around 38°C where the degree of supersaturation became 1.145, and the constant temperature was maintained until the degree of supersaturation reached 1.1 or less, and then cooling was performed again when the value of the degree of supersaturation of the supernatant was 1.091. When the temperature of the allulose solution was lowered to 25°C or less, a section where the degree of supersaturation gradually increased to 1.144 was shown, so crystallization was not terminated at a temperature of 20°C, and after maintaining for a certain period of time, and then reducing the degree of supersaturation to 1.110, the crystallization reaction was terminated. The mother liquid was removed by centrifugal dehydration and the crystals were washed with cooling water, and then dried to collect the allulose crystals.

The physical properties of the prepared allulose crystals were described in Table 1. The mean diameter of the allulose crystals of Example 2 was 378.4 µm, and the specific surface area value was 0.0203 m²/g. The appearance of the prepared allulose crystals was shown in FIG. 1b.

### Test Example 1. Identification of crystalline form of allulose

For the allulose crystals obtained in Comparative Examples 1 to 2 and Examples 1 to 2, X-ray diffraction analysis was performed according to the following analysis conditions, and major peaks of X-ray diffraction patterns of the allulose crystals were described in order of relative intensity (%) in Table 2.
Analysis instrument: D/MAX-2200 Ultima/PC
Manufacturer: Rigaku International Corporation (Japan)
X-ray sauce system target: sealed tube Cu
Tube voltage: 45 kV / Tube current: 200 mA
Scan range: 5 to 80° 2θ
Step size: 0.01°
Scan speed: 5°/min

**[Table 2]**

| Comparative Example 1 | | Comparative Example 2 | | Comparative Example 3 | | Comparative Example 4 | | Example 1 | | Example 2 | |
|---|---|---|---|---|---|---|---|---|---|---|---|
| Diffra ction angle | Relat ive inten sity (%) | Diffra ction angle | Relat ive inten sity (%) | Diffra ction angle | Relat ive inten sity (%) | Diffra ction angle | Relat ive inten sity (%) | Diffra ction angle | Relat ive inten sity (%) | Diffra ction angle | Relat ive inten sity (%) |
| 15.3 | 100 | 15.2 | 100 | 15.2 | 100 | 15.2 | 100 | 18.8 | 100 | 18.8 | 100 |
| 18.8 | 11.7 | 18.7 | 55.0 | 18.7 | 46.9 | 18.8 | 86.0 | 15.2 | 89.1 | 15.2 | 34.2 |
| 30.9 | 4.4 | 30.8 | 13.7 | 30.8 | 16.6 | 30.8 | 20.2 | 19.5 | 17.5 | 19.5 | 11.8 |
| 47.15 | 2.8 | 29.75 | 8.3 | 28.6 | 4.9 | 19.5 | 11.1 | 28.4 | 11.8 | 20.3 | 7.6 |

As shown in Table 2, the allulose crystals according to one embodiment of the present disclosure had a specific diffraction angle pattern different from conventional allulose crystals.

Specifically, in the X-ray diffraction pattern of the allulose crystals according to Comparative Example 1, the diffraction angles (2θ) of the major peaks were shown as 15.35°, 18.83°, 30.95°, and 47.15° in the order of the relative intensity. The XRD diffraction angle major peaks of the primary obtained allulose crystals and the crushed crystals in Comparative Example 1 were same, and this means that the crystal particles formed in the crystallization process maintain the same crystalline form even if the external shape of the crystal particles were changed through crushing and the like.

In addition, the diffraction angles (2θ) of the major peaks in the X-ray diffraction pattern of the allulose crystals according to Comparative Example 2 were shown as 15.2°, 18.8°, 30.8°, and 29.7° in the order of the relative intensity. Furthermore, the diffraction angles (2θ) of the major peaks in the X-ray diffraction pattern of the allulose crystals according to Comparative Example 3 were shown as 15.2°, 18.8°, 30.8°, and 28.3° in the order of the relative intensity. Moreover, the diffraction angles (20) of the major peaks in the X-ray diffraction pattern of the allulose crystals according to Comparative Example 4 were shown as 15.2°, 18.8°, 30.8°, and 19.5° in the order of the relative intensity.

On the other hand, in one embodiment of the present disclosure, in the X-ray diffraction pattern of the allulose crystals according to Example 1, the diffraction angles of the major peaks were shown as 18.8°, 15.2°, 19.5°, and 28.4°, and there was a difference in the relative intensity values of the major peaks, when compared with the X-ray diffraction pattern of conventionally prepared allulose crystals.

In addition, in the X-ray diffraction pattern of the allulose crystals according to Example 2 according to one embodiment of the present disclosure, the diffraction angles (2θ) of the major peaks were shown as 18.8°, 15.2°, 19.5°, and 20.3°. As same as Example 1, there was a difference in intensity values of the major peaks when compared to the X-ray diffraction pattern of conventionally prepared allulose crystals. In the X-ray diffraction analysis result of the allulose crystals, the diffraction angles (2θ) of peaks, the order according to the relative intensity of the diffraction angles of the peaks and the like affects the orientation property and angles of repose of crystals and the like, so the shape, structure and orientation of the allulose crystals may be different.

### Test Example 2. Analysis of appearance characteristics of prepared allulose crystals

The lightness (L value), redness (a value), yellowness (b value) and whiteness of the allulose crystals prepared in Comparative Examples 3 to 4 and Examples 1 to 2 were measured using a color difference meter (Spectro color meter SA-2000, Nippon Denshoku Industries co., Ltd.) and a whiteness measuring instrument (Whiteness Tester C-130, KETT electric laboratory), and described in Table 3.

**[Table 3]**

| Classification | Color difference value (color) | Whiteness |
|---|---|---|
| | L*, a*, b* | Whiteness index |
| Comparative Example 3 | 97.2, -0.14, 0.65 | 97.3 |
| Comparative Example 4 | 96,9, -0.11, 0.72 | 95.7 |
| Example 1 | 93.0, -0.07, 0.26 | 87.5 |
| Example 2 | 92.6, -0.06, 0.21 | 86.8 |

The whiteness shows higher value as closer to white color, and in general, sugar shows whiteness at a level of 80~86. In case in that a lot of fine powder are included or the particle surface is blunt, or in case of rice, depending on the cultivation environment, when starch particles are not densely accumulated and milky white rice kernels in which rice grains are loosely formed are generated, the whiteness value may further increase due to differences in light scattering. The crystals of Examples 1 and 2 showed whiteness at a similar level to sugar compared to Comparative examples 3 and 4, and showed differences in color values as lower yellowness (b value) being measured in the color difference value analysis, and the like. The high yellowness can be inferred that the mother liquid of the crystals may be attached to the crystal particles in the dehydration and washing process after crystallization, and when the crystal mother liquid remains in the particles even at a very small amount, it may induce browning of color or promote caking during long-term storage. The allulose crystals according to one embodiment of the present disclosure showed low yellowness, and this means that formation of crystal particles is uniformly achieved, so washing was smoothly performed during the dehydration process.

### Test Example 3. Analysis of particle characteristics of allulose crystals

For the allulose crystals obtained in Comparative examples 1 to 4 and Examples 1 to 2, the mean diameter and diameter distribution were measured using a diameter analysis equipment in a laser diffraction method, and the measured specific surface area was described in Table 1.
- Diameter analysis equipment: Laser Diffraction particle analyzer, Mastersizer 2000 (MALVERN Panalytical Ltd.)
- Dispersion Unit: Hydro 2000 MU (wet type)
- Dispersion solvent: Isopropyl alcohol

As shown in Table 1, the allulose crystals according to one embodiment of the present disclosure had a large size of a mean diameter of 230 µm or more and had a very low specific surface area of 0.04 m²/g or less.

### Test Example 4. Measurement of flowability of allulose crystals

Samples of the allulose crystals obtained in Comparative Examples 3 to 4 and Examples 1 to 2 were prepared by 150g each, and the angle of repose was measured using a measuring equipment of angle of repose in an automatic stirring type (manufacturer: K-one Nano., Ltd., model name: BT-200DA). The allulose crystal powder samples were passed through in a certain volume through a special funnel fixed at a certain height on a completely flat reference plate of the measuring equipment, and the angle of repose of the cone shape piled on the reference plate was measured, and the result was shown in Table 4. In addition, numerical values converting the angle of repose into a relative angle of repose based on 100% of angle of repose of sugar were shown in Table 4.

**[Table 4]**

| Classification | Angle of repose | Relative angle of repose compared to sugar (%) |
|---|---|---|
| Comparative Example 3 | 57.8° | 144.5% |
| Comparative Example 4 | 49.6° | 124% |
| Example 1 | 43.4° | 108.5% |
| Example 2 | 42.3° | 105.75% |

As shown in Table 4, it was confirmed that the allulose crystals according to one embodiment of the present disclosure had improved flowability of particles compared to conventional allulose crystals, and had an angle of repose at a similar level to sugar, so convenience of use was increased in cases such as inputting crystal products or line transferring or the like.

### Test Example 5. Comparison of hygroscopicity of allulose crystals

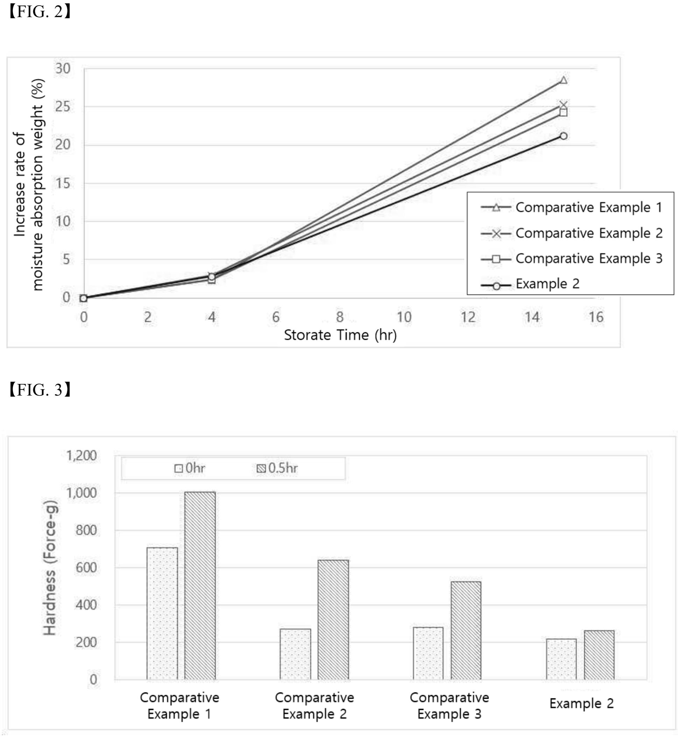

Samples of the allulose crystals prepared in Comparative Examples 1 to 3 and Example 2 were accurately weighed by 10g each, and prepared by spreading them evenly on a weighing dish. By storing under conditions of a temperature of 25°C and a humidity of 70% in a thermos-hygrostat, the weights of the samples were measured over time, and the weights increased by hygroscopicity compared to the initial weights were calculated and shown in percentage in Table 5 and FIG. 2.

**[Table 5]**

| Sample | Weight increase rate compared to initial by storage time under hygroscopic conditions (%) | |
|---|---|---|
| | 4hr | 15hr |
| Comparative Example 1 | 2.4 | 28.5 |
| Comparative Example 2 | 2.9 | 25.3 |
| Comparative Example 3 | 2.4 | 24.2 |
| Example 2 | 2.7 | 21.2 |

As shown in Table 5 and FIG. 2, the allulose crystals according to one embodiment of the present disclosure have improved hygroscopicity, and therefore, it can maintain stability in product packaging and distribution processes.

### Test Example 6. Measurement of caking hardness of allulose crystals

25g of the allulose crystal samples prepared in Comparative Examples 1 to 3 and Example 2 were placed on the same aluminum dish with an even surface without applying external pressure, respectively. While storing by maintaining thermos-hygrostat conditions of the temperature of 40°C and relative humidity of 70%, each sample was taken out at 0 hour and 30 minutes of storage, and left as it was at a room temperature (25°C) for 30 minutes to cool heat, and then a weight with the same weight (25g) as the sample was placed on the sample surface for 30 minutes in the same way and caking on the allulose crystal surface was induced. Specifically, by applying pressure on the surface using a weight with the same weight as the allulose crystal sample, a caking phenomenon, which could occur when the allulose crystals were loaded and stored, was to be reproduced. The caking hardness measurement conditions were as follows:
- Equipment name: Texture analyzer TAXTplus (stable micro systems)
- Cylinder probe: 25mmφ Perspex
- Test speed: 2mm/sec
- Trigger force: 5g

The caking hardness over time of each sample was shown in Table 6 and FIG. 3.

**[Table 6]**

| Sample | Surface caking hardness (Hardness, force-g) | | |
|---|---|---|---|
| | 0 h | 0.5 hr | Hardness increase rate (%) |
| Comparative Example 1 | 708.7 | 1002.7 | 41.48 |
| Comparative Example 2 | 272.5 | 640.2 | 134.94 |
| Comparative Example 3 | 282.0 | 524.6 | 86.03 |
| Example 2 | 218.8 | 260.3 | 18.97 |

As shown in Table 6 and FIG. 3, the surface caking hardness of the allulose crystals of Example 2 was the lowest, and the change in surface caking harness was the least even as the time exposed to high temperature and hygroscopic conditions. On the other hand, the allulose crystals of Comparative Examples 1 to 3, in which there were differences in specific surface area with particles, showed a significant increase rate of caking hardness as well as the initial caking hardness. Therefore, when stored for a long period of time, as the effect by the external environment is large, and the storage loadage is large, the surface caking of the product can be accelerated, and it was confirmed that in the allulose crystal samples according to one embodiment of the present disclosure, the surface caking degree was alleviated even under harsh storage conditions and thus the storge stability was improved. Therefore, it can provide significant help in improvement of storage stability during packaging, storage, transportation, and distribution of products industrially.

### Test Example 7. Sensory evaluation of allulose crystals

For sensory evaluation, the allulose crystal samples prepared in Comparative Examples 1 to 3 and Example 2 were prepared in each same amount, and 15 panels with a high understanding of sensory testing and 10 years or more of sensory testing experience were selected, and samples were individually provided to sensory testers and evaluated using the 5-point method. For sweetness and refreshing sensation, the higher the score shows that the sweetness or refreshing sensation is "better" or "stronger", and for bitterness and off-taste/off-smell, the lower the score shows that the bitterness or off-taste/off-smell is weaker, so is "better", and the higher the score shows that the bitterness or off-taste/off-smell is stronger, so is "worse".

Each sample was expressed as a three-digit number derived randomly using a random number table, and the sample presentation order was always determined randomly, and lukewarm water to rinse mouths was provided to testers together. The sensory testing room was maintained at a certain temperature (25±1°C) without a smell.

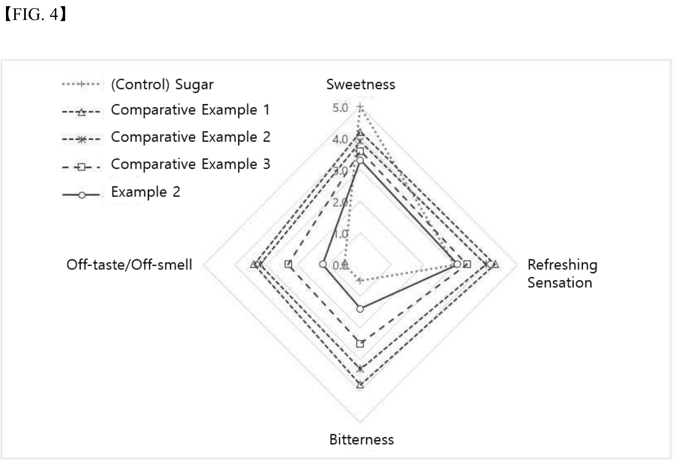

The evaluation content and method were evaluated with items of sweetness, refreshing sensation, bitterness and off-taste and off-smell as sensory characteristics of the allulose crystals, and control sugar was provided together as a reference sample, and thereby, the reference values by sensory item were presented to compare and evaluate the sensory items when compared with sugar using a 15cm linear scale. The numerical values of the sensory evaluation result were shown in Table 7 below, and the sweetness profile graph of the sensory evaluation result was shown in FIG. 4.

**[Table 7]**

| Intensity | Sweetness | Refreshing sensation | Bitterness | Off-taste/off-smell | Overall satisfaction |
|---|---|---|---|---|---|
| (Control) sugar | 5.0 | 3.0 | 0.5 | 0.5 | 5.0 |
| Comparative Example 1 | 4.2 | 4.3 | 3.8 | 3.4 | 3.0 |
| Comparative Example 2 | 3.9 | 4.0 | 3.3 | 3.2 | 3.2 |
| Comparative Example 3 | 3.6 | 3.4 | 2.5 | 2.3 | 3.5 |
| Example 2 | 3.3 | 3.1 | 1.4 | 1.2 | 4.6 |

As a result of the sensory evaluation, the sweetness was analyzed to be lower than sugar in the same way as the known degree of sweetness, but the sweetness profile in Example 2 was shown similarly to sugar than the Comparative Example samples, and in particular, the Comparative Example samples with a large surface area of the allulose crystal particles had a greater intensity felt sensually and had worse sensory characteristics for bitterness and off-taste and off-smell than sugar. On the other hand, it was confirmed that the allulose crystals according to one embodiment of the present disclosure had a small specific surface area and had a very similar texture to sugar, so the intensity of off-taste and off-smell was felt the least, and the bitter was also improved.

## Claims

1. An allulose crystal, having an X-ray powder diffraction pattern comprising peaks at positions of 2θ diffraction angles of 18.8±0.5°, 15.2±0.5°, and 19.5±0.5° in X-ray powder diffraction (XRD) analysis.

2. The allulose crystal according to claim 1, having an X-ray powder diffraction pattern comprising peaks at positions of 2θ diffraction angles of 18.8±0.5°, 15.2±0.5°, 19.5±0.5°, and 28.4±0.5° in X-ray powder diffraction (XRD) analysis.

3. The allulose crystal according to claim 1, having an X-ray powder diffraction pattern comprising peaks at positions of 2θ diffraction angles of 18.8±0.5°, 15.2±0.5°, 19.5±0.5°, and 20.3±0.5° in X-ray powder diffraction (XRD) analysis.

4. The allulose crystal according to claim 1, wherein the peaks are peaks having a relative intensity of 5% or more.

5. The allulose crystal according to claim 1, wherein the allulose crystal has a specific surface area of 0.04 m²/g or less.

6. The allulose crystal according to claim 1, wherein the allulose crystal has D[4,3] (volume-average particle diameter) of 230 µm or more.

7. The allulose crystal according to claim 1, wherein the allulose crystal has an angle of repose of 120% or less compared to the angle of repose of sugar.

8. The allulose crystal according to claim 1, wherein the allulose crystal has an angle of repose of 48° or less.

9. The allulose crystal according to claim 1, wherein the allulose crystal has hygroscopic rate of 24% or less when stored for 15 hours under conditions of a temperature of 25°C and a relative humidity of 70%.

10. The allulose crystal according to claim 1, wherein the allulose crystal has an increase rate of hardness of 40% or less after being stored under conditions of a temperature of 40°C and a relative humidity of 70% for 0.5 hours, left at a temperature of 25°C for 30 minutes, and applied a pressure of the same weight as the allulose crystal for 30 minutes.

11. The allulose crystal according to claim 1, wherein the allulose crystal is crystallized under a condition that the total specific surface area of all seeds contained in the crystallization reaction system is 0.05 m²/100g or less.

12. The allulose crystal according to claim 1, wherein the allulose crystal is crystallized under a condition that the degree of supersaturation of allulose solution is 1.15 or less.

13. A sweetener composition comprising the allulose crystal according to any one of claim 1 to claim 12.

14. A method of preparing the allulose crystal according to any one of claim 1 to claim 12, comprising
a step of adding seeds to allulose solution so that the total specific surface area of all seeds contained in the crystallization reaction system is 0.05 m²/100g or less; and
a step of forming an allulose crystal while maintaining the degree of supersaturation of the allulose solution at 1.15 or less.

15. The method of preparing according to claim 14, wherein the step of forming an allulose crystal is performed by cooling a temperature of the allulose solution at a speed of -0.5°C/hr or less.

16. The method of preparing according to claim 14, wherein controlling the speed of cooling of the allulose solution is performed so as to maintain the degree of supersaturation of the allulose solution at 1.15 or less.

17. The method of preparing according to claim 16, wherein the controlling the speed of cooling of the allulose solution comprises temporarily stopping cooling of the allulose solution.

18. The method of preparing according to claim 16, wherein the controlling the speed of cooling of the allulose solution is temporarily stopping cooling of the allulose solution at a temperature within a range of 15 to 45°C to maintain the temperature of the allulose solution constantly.

19. The method of preparing according to claim 14, wherein the step of forming the allulose crystal comprises temporarily stopping cooling until the degree of supersaturation of the allulose solution reach 1.1 or less, when the degree of supersaturation of the allulose solution exceeds 1.15, to maintain the temperature of the allulose solution constantly.

20. The method of preparing according to claim 14, wherein the step of forming the allulose crystal is performed by starting cooling of the allulose solution at the degree of supersaturation of the allulose solution of more than 1 to 1.15 or less.

21. The method of preparing according to claim 14, wherein the step of forming the allulose crystal is performed by starting cooling of the allulose solution at a temperature of the allulose solution of 25 to 50°C.

22. The method of preparing according to claim 14, wherein the step of forming the allulose crystal is performed by terminating cooling of the allulose solution at a temperature of the allulose solution of 15 to 25°C.
